# NEUE EUROPÄISCHE PATENTSCHRIFT

(11) **EP 0 295 552 B2**
(45) Veröffentlichungstag und Bekanntmachung des Hinweises auf die Entscheidung über den Einspruch: **13.01.1999**
(45) Hinweis auf die Patenterteilung: 18.03.1992
(21) Anmeldenummer: 88109090.6
(22) Anmeldetag: 08.06.1988
(51) Int. Cl.: C07C 69/533, C07C 67/333

(54) **Verfahren zur Herstellung von Pentensäureestern**
Method for the production of pentenoic acid esters
Procédé pour la fabrication d'esters de l'acide penténoique

(30) Priorität: 15.06.1987 DE 3719935
(43) Veröffentlichungstag der Anmeldung: 21.12.1988
(73) Patentinhaber: BASF Aktiengesellschaft, 67063 Ludwigshafen (DE)
(72) Erfinder: Naeumann, Fritz, Dr., D-6800 Mannheim 1 (DE); Hoelderich, Wolfgang, Dr., D-6710 Frankenthal (DE); Merger, Franz, Dr., D-6710 Frankenthal (DE)

(56) Entgegenhaltungen:
- EP-A- 81 090
- EP-A- 131 860
- DE-B- 1 917 244
- US-A- 3 880 913
- US-A- 4 035 408
- US-A- 4 517 400
- JOURNAL OF THE AMERICAN CHEMICAL SOCIETY, Band 90, Nr. 1, 3. Januar 1968, S. 94-97, American Chemical Society, Columbus, Ohio, USA; J. TSUJI et al, "Organic Syntheses by Means of Noble Metal Compounds. XXXIV. Carbonylation and Decarbonylation Reactions Catalyzed by Palladium"
- D.W. Breck, Zeolite molecular sieves: structure, chemistry and use, John Wiley & Sons, 1974, page 92
- J. Am. Chem. Soc., 1984, 106, 6092-6093, M.L. Brent et al, Silicoaluminophosphate molecular sieves

## Beschreibung

Bei der Hydroformylierung von Pentensäureestern, wie sie beispielsweise in den Europäischen Patentschriften 31 100 und 125 567 beschrieben wird. erhält man ein Gemisch aus 5-, 4- und 3-Formylvaleriansäureestern in wechselnder Zusammensetzung. Da für die Weiterverarbeitung jeweils nur eines der Isomeren erwünscht ist, z.B. 5-Formylvaleriansäureester, sind die übrigen Isomeren unerwünschte Nebenprodukte.

Aus der US-PS 4 517 490 ist bereits bekannt, daß man bei der Dehydrocarbonylierung eines Gemisches aus geradkettigen und verzweigten Aldehyden in Gegenwart von Zeolithen, die Edelmetalle wie Platin, Palladium oder Rhodium enthalten, Olefine erhält. Hierbei werden jedoch nur selektiv die geradkettigen Aldehyde dehydrocarbonyliert, während die verzweigten Aldehyde erhalten bleiben. So wird z.B. bei der Behandlung eines Gemisches aus n- und i-Butyraldehyd n-Butyraldehyd mit einem Umsatz von 39 bis 56 % zu Propylen umgesetzt, während der gleichfalls enthaltene i-Butyraldehyd praktisch nicht reagiert. In der deutschen Patentschrift 19 17 244 wird auch ein Verfahren zur Dehydrocarbonylierung von Isobutyraldehyd beschrieben, wobei man Rhodium und/oder Platin enthaltende Träger-Katalysatoren verwendet, Unterhalb von 250° C sinkt jedoch der Umsatz beträchtlich ab, so daß der eingesetzte Isobutyraldehyd zu einem großen Teil unverändert zurückgewonnen wird. Ein Hinweis, wie Formylvaleriansäureester wiederum in Pentenester umgewandelt werden, wird nicht gegeben.

US-A 3,880,913 beschreibt die Dehydroformylierung von isomeren Acetoxybutyraldehyden, die als unerwünschte Nebenprodukte bei der Hydroformylierung von Allylacetat oder 1-Propenylacetat zu 4-Acetoxybutyraldehyd anfallen, bei 120 bis 250°C in einer nichtoxidierenden Atmosphäre an einem im wesentlichen neutralen Edelmetallkatalysator zu Allylacetat, 1-Propenylacetat oder einer Mischung daraus.

Eine analoge Übertragung der für Butyraldehyd gewonnenen Erkenntnisse auf Formylvaleriansäureester war insofern nicht angezeigt, als aus der Europäischen Patentanmeldung 81 090 bekannt war, daß 4-Formylbuttersäureester beim Erhitzen in Gegenwart von Zeolithen wie Montmorillonit auf eine Temperatur von 200 bis 350° C unter Ringschluß zu Dihydropyronen umgesetzt werden.

Es war deshalb die technische Aufgabe gestellt ein Verfahren zur Herstellung von Pentensäureestern aus Formylvaleriansäureestern zur Verfügung zu stellen, bei dem man die Ringbildung zu Dihydropyronen vermeidet, das mit hohen Umsätzen und Selektivitäten verlauft und die Hydrierung der Pentensäureester zu Valeriansäureestern minimiert wird.

Diese Aufgabe wird gelöst in einem Verfahren zur Herstellung von Pentensäureestern, wobei man Formylvaleriansäureester in Gegenwart von Zeolithen oder Phosphaten wobei die Zeolithe oder Phosphate einen Gehalt an mindestens einem Metall der VIII. Nebengruppe des periodischen Systems haben, ausgewählt aus der Gruppe Aluminiumphosphat, Siliciumaluminiumphosphat, Boraluminiumphosphat, Siliciumeisenaluminiumphosphat, Cerphosphat, Zirkonphosphat, Borphosphat, Eisenphosphat und Strontiumphosphat oder deren Gemische als Katalysatoren auf eine Temperatur von 50 bis 400°C erhitzt.

Das neue Verfahren hat den Vorteil, daß es mit hohen Umsätzen und hohen Selektivitäten verläuft. Weiter hat das neue Verfahren den Vorteil, daß die Hydrierung der erzeugten Pentensäureester zu unerwünschten Valeriansäureestern minimiert wird und die Ringbildung zu Dihydropyronen vermieden wird.

Im Hinblick auf die in der Europäischen Patentanmeldung 81 090 beschriebene Ringbildung von 4-Formylbuttersäureestern zu Dihydropyronen war zu erwarten, daß sich verzweigte Formylvaleriansäureester ebenfalls unter Ringbildung zu Dihydropyronen umsetzen.

Im allgemeinen geht man von Formylvaleriansäureestern aus, die sich von Alkanolen mit 1 bis 12 Kohlenstoffatomen, Cycloalkanolen mit 5 bis 8 Kohlenstoffatomen, Aralkanolen mit 7 bis 10 Kohlenstoffatomen oder Phenolen oder Naphtholen ableiten. Bevorzugt verwendet man Formylvaleriansäurealkylester, insbesondere von Alkanolen mit 1 bis 4 Kohlenstoffatomen. Geeignete Verbindungen sind z.B. jeweils die Methyl-, Ethyl-, Propyl-, Isopropyl-, Butyl-, Hexyl-, Nonyl-, Dodecyl-, Cyclopentyl-, Cyclohexyl-, Benzyl- oder

Phenylester der 5-, 4- oder 3-Formylvaleriansäure. Besondere technische Bedeutung hat die Dehydrocarbonylierung der entsprechenden 4- und 3-Formylvaleriansäureester erlangt. Ein typisches Ausgangsgemisch enthält beispielsweise 50 bis 80 Gew.-% 4-Formylvaleriansäureester, 20 bis 40 Gew.-% 3-Formylvaleriansäureester und bis zu 10 Gew.% 5-Formylvaleriansäureester.

Die Umsetzung wird bei einer Temperatur von 50 bis 400°C durchgeführt. Vorteilhaft hält man eine Temperatur von 60 bis 350°C, insbesondere 70 bis 280°C ein. Im allgemeinen wird die Reaktion unter Atmosphärendruck durchgeführt. Es ist jedoch auch möglich schwach verminderten oder schwach erhöhten Druck, z.B. bis zu 20 bar, anzuwenden.

Vorteilhaft wird die Umsetzung unter Mitverwendung von molekularem Sauerstoff oder molekularen Sauerstoff enthaltenden Gasen, die vorteilhaft einen Gehalt an molekularem Sauerstoff von 5 bis 20 Vol.% haben, durchgeführt. Geeignet ist beispielsweise Luft, gegebenenfalls unter Zusatz von Inertgasen, wie Stickstoff der zugleich als Trägergas wirkt. Vorteilhaft wendet man je Mol Formylvaleriansäureester 0,05 bis 10, insbesondere 0,2 bis 3 Mol molekularen Sauerstoff an. Durch die Mitverwendung von molekularem Sauerstoff wird die Ausbeute an Pentenestern erhöht und die Lebensdauer der Katalysatoren verlängert.

Die Mitverwendung von molekularem Sauerstoff war an sich nicht angezeigt, da aus der europäischen Patentanmeldung 131 860 bekannt war, daß Formylvaleriansäureester bereits bei relativ niedrigen Temperaturen, z.B. unter 100°C, nahezu quantitativ zu den entsprechenden Dicarbonsäurehalbestern oxidiert werden.

Ferner hat es sich als vorteilhaft erwiesen die Umsetzung unter Mitverwendung von unter Reaktionsbedingungen inerten Verdünnungsmitteln durchzuführen. Geeignete Verdünnungsmittel sind beispielsweise Wasser, weiterhin Alkanole mit 1 bis 6 Kohlenstoffatomen, wie Methanol, Ethanol, Butanol oder Hexanol, Cycloalkanole mit 5 oder 6 Kohlenstoffatomen wie Cyclopentanol oder Cyclohexanol, ferner Ether wie Dioxan oder Tetrahydrofuran, darüber hinaus chlorierte Kohlenwasserstoffe wie Methylenchlorid, Chloroform, 1,2-Dichlorethan sowie aliphatische, cycloaliphatische oder aromatische Kohlenwasserstoffe wie Benzol, Toluol, Cyclohexan oder Paraffine. Es hat sich bewährt wenn man je Mol Formylvaleriansäureester 0,1 bis 50, insbesondere 0,5 bis 20 Mol Verdünnungsmittel anwendet. Besonders bevorzugte Verdünnungsmittel sind Wasser und Alkanole mit 1 bis 6 Kohlenstoffatomen oder deren Gemische.

Das erfindungsgemäße Verfahren wird in Gegenwart von Zeolithen oder mindestens einem Phosphat ausgewählt aus der Gruppe Aluminiumphosphat, Siliciumaluminiumphosphat, Boraluminiumphosphat, Siliciumeisenaluminiumphosphat, Cerphosphat, Zirkonphosphat, Borphosphat, Eisenphosphat und Strontiumphosphat, durchgeführt, die einen Gehalt an mindestens einem Metall der VIII. Nebengruppe der Periodischen Systems wie Palladium, Platin, Ruthenium, Rhodium, Osmium, Iridium, Eisen, Kobalt, Nickel, insbesondere Edelmetalle haben. Besonders bevorzugt enthalten die Katalysatoren jeweils zwei Metalle ausgewählt aus der Gruppe Ruthenium, Rhodium, Palladium, Platin, Iridium, Osmium. Gute Ergebnisse erhält man auch wenn man jeweils mindestens ein Edelmetall wie Ruthenium, Rhodium, Palladium, Platin, Iridium oder Osmium, mit mindestens einem Metall oder Eisengruppe, z.B. Eisen, Kobalt oder Nickel, kombiniert.

Vorzugsweise beträgt der Gehalt an Metallen der VIII. Nebengruppe des Periodischen Systems 0,01 bis 10 Gew.%, vorzugsweise 0,05 bis 5 Gew.%, insbesondere 0,05 bis 2 Gew.%, berechnet als Metall und bezogen auf die Summe von Zeolith oder Phosphat und Metall.

Darüber hinaus ist es vorteilhaft wenn die verwendeten Zeolithe und Phosphate zusätzlich einen Gehalt an mindestens einem Element der I. bis VII. Nebengruppe des Periodischen Systems, z.B. Zink, Kupfer, Silber, Titan, Vanadium, Chrom, Molybdän, Wolfram, Mangan oder Rhenium haben Der Gehalt an Elementen der I. bis VII. Nebengruppe des Periodischen Systems beträgt vorteilhaft 0,05 bis 2 Gew.%, berechnet als Metall und bezogen auf die Summe von Zeolithen oder Phosphaten und katalytisch aktive Metalle.

Die erfindungsgemäß verwendeten Zeolithe haben eine kristalline hochgeordnete Struktur mit einem dreidimensionalen Netzwerk von Me(IV)O₄- und Me(III)O₄-Tetraethern, die durch gemeinsame Sauerstoffatome verbunden sind. Die Elektrovalenz ist durch Einschluß von Kationen, z.B. von Alkali- oder Wasserstoffionen ausgeglichen. Als dreiwertige Metalle (Me(III) eignen sich beispielsweise Bor, Gallium, Eisen, Chrom, Vanadium, Arsen, Antimon, Wismuth, Beryllium, insbesondere Aluminium oder deren Gemische. Während als vierwertige Metalle (MeIV) z.B. Germanium, Titan, Zirkon, Hafnium, insbesondere Silicium oder deren Gemische enthalten sind.

Vorteilhafte Zeolithe sind Alumino-, Boro-, Eisen-, Beryllium-, Gallium-, Chrom-, Arsen-, Antimon- und Wismuth-Silikatzeolithe oder deren Gemische, ferner Alumino-, Boro-, Gallium- und Eisengermanatzeolithe oder deren Gemische.

Besonders bewährt haben sich Zeolithe mit Faujasit-, Erionit- oder Chabasit-Struktur, Y-, X- oder L-Zeolithe, ferner Zeolithe mit Pentasilstruktur. Hiervon sind Zeolithe mit Pentasilstruktur besonders bevorzugt. Besonders hervorgehoben seien Alumino-, Boro- und Eisensilikatzeolithe mit Pentasilstruktur.

Aluminiumsilikatzeolithe werden z.B. aus einer Aluminiumverbindung, vorzugsweise Aluminiumhydroxid oder Aluminiumsulfat und einer Siliciumkomponente, vorzugsweise hochdispersem Siliciumdioxid, in wäßriger Aminlösung, insbesondere in Polyaminen wie 1,6-Hexandiamin, 1,3-Propandiamin oder Triethylentetramin mit oder insbesondere ohne Alkali- oder Erdalkalizusatz bei einer Temperatur von 100 bis 220°C unter autogenem Druck hergestellt. Hierzu gehören auch die isotaktischen Zeolithe, wie sie beispielsweise in den europäischen Patentanmeldungen 34 727 und 46 504 beschrieben werden. So erhaltene Aluminosilikatzeolithe enthalten je nach Wahl der eingesetzten Verbindungen ein SiO₂/Al₂O₃-Verhältnis von 10 bis 40.000. Aluminosilikatzeolithe mit Pentasilstruktur sind auch in etherischem Medium wie Diethylenglykoldimethylether, in alkoholischem Medium wie Methanol oder Butanol, ferner in Wasser erhältlich. Zu den erfindungsgemäß verwendbaren siliciumreichen Zeolithen mit einem Verhältnis SiO₂/Al₂O₃ ≧ 10 gehören auch die verschiedenen ZSM-Typen, der Ferrierit und der NU-1. Solche Zeolithe werden beschrieben in US 3 702 886, US 3 832 449, US 4 076 859; (Ferrierit) EP 12 473 (NU-1) US 4 060 590. Hierzu gehört auch der Silicalit® (US-PS 4 061 724).

Borosilikatzeolithe erhält man z.B. durch Umsetzen von Borsäure mit hochdispersem Siliciumdioxid in wäßriger Aminlösung, insbesondere 1,6-Hexandiamin, 1,3-Propandiamin oder Triethylentetramin mit und insbesondere ohne Alkali- oder Erdalkalizusatz bei einer Temperatur von 90 bis 200°C unter autogenem Druck. Hierzu gehören auch die isotaktischen Zeolithe wie sie in den EP-Anmeldungen 34 727 und 46 494 beschrieben werden. Geeignete Reaktionsmedien sind anstatt wäßrigen Aminlösungen auch etherische Lösungen, z.B. Diethylenglykoldimethylether oder alkoholische Lösungen, z.B. Hexandiol-1,6.

Eisensilikatzeolithe erhält man z.B. durch Umsetzen einer Eisenverbindung, vorzugsweise Eisen-III-sulfat mit hochdispersem Siliciumdioxid in einer wäßrigen Aminlösung, insbesondere 1,6-Hexandiamin mit oder ohne Alkali- oder Erdalkalizusatz bei einer Temperatur von 100 bis 200°C unter autogenem Druck.

So hergestellte Alumino-, Boro- und Eisensilikatzeolithe werden im allgemeinen nach ihrer Isolierung, Trocknen bei einer Temperatur von 108 bis 160°C, vorzugsweise 110 bis 130°C und Calcinieren bei einer Temperatur von 450 bis 550°C, vorzugsweise 480 bis 520°C, mit einem Bindemittel im Verhältnis von 90:10 bis 40:60 Gew.-Teilen zu Strängen oder Tabletten verformt. Als Bindemittel eignen sich Aluminiumoxide, insbesondere Boehmit, amorphe Aluminiumsilikate mit einem SiO₂/Al₂O₃-Verhältnis von 25:75 bis 90:5, bevorzugt 75:25, ferner Siliciumdioxid, insbesondere hochdisperses Siliciumdioxid, sowie Gemische aus hochdispersem Siliciumdioxid und hochdispersem Aluminiumoxid, Titandioxid, Zirkondioxid oder Ton. In der Regel werden die Extrudate oder Preßlinge nach der Verformung bei einer Temperatur von 110 bis 130°C, z.B. 16 Stunden getrocknet und anschließend bei einer Temperatur von 450 bis 550°C, z.B. 16 Stunden calciniert.

Man erhält auch vorteilhafte Katalysatoren wenn man Alumino- oder Borosilikatzeolithe direkt nach der Trocknung verformt und erst nach der Verformung calciniert. Die hergestellten Alumino- und Borosilikatzeolithe können in reiner Forme ohne Binder als Stränge oder Tabletten eingesetzt werden wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silicoester oder Graphit oder deren Gemische verwendet werden.

Liegen der Zeolithe aufgrund ihrer Herstellung z.B. in der Na-Form vor, so kann diese durch Ionenaustausch, z.B. mit Ammoniumionen und anschließende Calcinierung oder durch Behandlung mit Säuren vollkommen oder partiell in die H-form überführt werden. Durch Ionenaustausch können auch andere Alkali- oder Erdalkalimetallionen, z.B. Lithium, Caesium, Kalium oder Magnesium, Calcium, Strontium, Barium, eingebaut werden.

Eine weitere Möglichkeit der Modifizierung besteht darin, daß man Zeolithe verformt oder unverformt einer Behandlung mit Säuren wie Salzsäure, Flußsäure und Phosphorsäure und/oder Wasserdampf unterwirft.

Eine besondere Ausführungsform für die Säurebehandlung besteht darin, daß man Zeolithe vor ihrer Verformung bei erhöhter Temperatur mit Flußsäure, die im allgemeinen als 0,001 bis 2 n, vorzugsweise 0,05 bis 0,5 n Flußsäure eingesetzt wird, behandelt, beispielsweise durch Erhitzen unter Rückfluß für einen Zeitraum von z.B. 0,5 bis 5, vorzugsweise 1 bis 3 Stunden. Nach dem Abtrennen z.B. durch Abfiltrieren werden die Zeolithe gewaschen und anschließend z.B. bei einer Temperatur von 100 bis 160°C getrocknet und anschließend bei einer Temperatur z.B. von 450 bis 600°C calciniert.

Nach einer anderen bevorzugten Ausführungsform für die Säurebehandlung werden die Zeolithe nach der Verformung mit Bindemitteln bei erhöhter Temperatur z.B. 50 bis 90°C, insbesondere 60 bis 80°C für einen Zeitraum z.B. von 0,5 bis 5 Stunden mit vorzugsweise 12 bis 20 gew.%iger Salzsäure behandelt. Nach dem Abfiltrieren werden die Zeolithe zweckmäßig mit Wasser ausgewaschen und bei einer Temperatur von 100 bis 160°C getrocknet und anschließend bei einer Temperatur von 450 bis 600°C calciniert. Es ist auch möglich, zunächst eine Behandlung mit Flußsäure durchzuführen und anschließend eine Behandlung mit Salzsäure.

Metalle der VIII. Nebengruppe und gegebenenfalls zusätzlich Elemente der I. bis VII. Nebengruppe werden vorteilhaft durch Ionenaustausch oder durch Tränken der Zeolithe aufgebracht.

Ein Ionenaustausch der in der H-Form, Ammoniumform oder Alkaliform vorliegenden Zeolithe wird z.B. so vorgenommen, daß man Zeolitstränge oder Pellets in einer Kolonne vorlegt und darüber eine ammoniakalische Metallsalzlösung, z.B. Platinnitrat bei erhöhter Temperatur, z.B. 30 bis 80°C für einen Zeitraum von 15 bis 20 Stunden im Kreislauf führt. Anschließend wird mit Wasser ausgewaschen und wie oben ausgeführt, getrocknet und caliciniert.

Nach einer anderen Arbeitsweise werden Metallsalze, insbesondere Nitrate, die beim Erhitzen Metalloxide ergeben, als wäßrige Lösung, z.B. Hexachlorplatinat, Platinnitrat, Kupfernitrat, Nickelnitrat oder Cernitrat durch Tränken auf den Zeolith aufgebracht und gegebenenfalls überschüssiges Wasser verdampft. Danach wird der getränkte Zeolith wie üblich getrocknet und kalziniert. Der Tränkvorgang kann mehrmals hintereinander vorgenommen werden um den gewünschten Metallgehalt einzuzstellen. Solche Metalle enthaltenden Zeolithe werden vorteilhaft vor der Verwendung, z.B. bei einer Temperatur von 150 bis 300°C und gegebenenfalls unter einem Druck von 0,2 bis 2 bar mit Wasserstoff reduziert.

Von den eingangs erwähnten Phosphatkatalysatoren eignen sich insbesondere diejenigen die hydrothermal erhältlich sind. Besondere Bedeutung haben Aluminiumphosphate (APO) oder Siliciumaluminiumphosphate (SAPO) erlangt.

Geeignete Aluminiumphosphate (APO) sind z.B. APO-5, APO-9, APO-11, APO-12, APO-14, APO-21, APO-25, APO-31 und APO-33. Solche Aluminiumphosphate werden beispielsweise beschrieben in S.T. Wilson, B.M. Lok, C.A. Messina, E.A. Flanigen, Seiten 97-109 in Proc. 6th Fut. Zeolithe Conf., Reno 1983, Herausgeber D.M. Olson, A. Bisio.

ALPO₄-5 (APO-5) erhält man beispielsweise, in dem man o-Phosphorsäure mit Pseudoboehmit in Wasser homogen mischt, Tetrapropylammoniumhydroxid zugibt und anschließend für einen Zeitraum von 20 bis 60 Stunden auf eine Temperatur von 100 bis 200°C unter autogenem Druck erhitzt. Das so erhältliche Aluminiumphosphat wird durch Filtration isoliert, z.B. bei einer Temperatur von 100 bis 160°C getrocknet und anschließend bei einer Temperatur von 450 bis 550°C kaliziniert.

ALPO₄-9 (APO-9) erhält man beispielsweise durch Umsetzen von o-Phosphorsäure mit Pseudoboehmit in wäßriger Lösung von 1,4-Diazobicyclo(2,2,2)-Octan bei einer Temperatur von 100 bis 200°C für einen Zeitraum von 200 bis 400 Stunden unter autogenem Druck. Verwendet man anstelle von 1,4-Diazabicyclo(2,2,2)-Octan Ethylendiamin, so erhält man APO-12. Verwendet man bei analoger Arbeitsweise eine wäßrige Lösung von Pyrrolidon und wendet eine Temperatur von 150 bis 200°C an für einen Zeitraum von 50 bis 200 Stunden, so erhält man APO-21.

Geeignete Siliciumaluminiumphosphate (SAPO) sind beispielsweise SAPO-5, SAPO-11, SAPO-31 und SAPO-34. Solche Siliciumaluminiumphosphate werden beispielsweise beschrieben in E. M. Flanigen, B.M. Lok, R.L. Patton, S.T. Wilson, Pure Appl. Chem., 58 (1986), 1351-1358.

SAPO-5 erhält man beispielsweise durch Mischen von Siliciumdioxid, suspendiert in wäßriger Tetrapropylammoniumhydroxidlösung mit einer wäßrigen Suspension von Pseudoboehmit und o-Phosphorsäure und Umsetzen bei 150 bis 200°C für einen Zeitraum von 20 bis 200 Stunden unter autogenem Druck. Anschließend wird das abfiltrierte Pulver zweckmäßig bei einer Temperatur von 110 bis 160°C getrocknet und bei einer Temperatur von 450 bis 550°C calciniert.

Geeignete Siliciumaluminiumphosphate sind auch ZYT-5, ZYT-6, ZYT-7, ZYT-9, ZYTL-11 und ZYT-12. Solche Siliciumaluminiumphosphate werden beispielsweise beschrieben in Japanische Patentanmeldung 59217-619.

Geeignete Aluminiumphosphate erhält man auch durch Fällung. Beispielsweise wird hierbei zu einer wäßrigen Diammoniumhydrogenphosphatlösung eine wäßrige Lösung von Aluminiumnitrat langsam zugegeben und dabei der pH-Wert durch gleichzeitiges Zuführen von z.B. 25 %iger Ammoniaklösung auf einen Wert von pH 6 bis 8 gehalten. Der entstandene Niederschlag wird 12 Stunden nachgerührt, abgesaugt und ausgewaschen und anschließend bei einer Temperatur z.B. 100 bis 160°C für einen Zeitraum von 2 bis 24 Stunden getrocknet.

Borphosphate erhält man beispielsweise durch Mischen und Kneten von konzentrierter Borsäure und Phosphorsäure und anschließendes Trocknen und calcinieren in Inertgas-, Luft- oder Dampfatmosphäre bei einer Temperatur von 250 bis 650°C, vorzugsweise 300 bis 500°C.

Zur Modifizierung der Phosphate mit Metallen der VIII. Nebengruppe und gegebenenfalls mit Elementen der I. bis VII. Nebengruppe werden geeignete Verbindungen wie Nitrate, die beim Erhitzen in die Oxide übergehen, durch Tränken aufgebracht und anschließend getrocknet und caliciniert.

Die vorbeschriebenen Katalysatoren werden in der Regel als 2 bis 4 mm Stränge als Tabletten mit 3 bis 5 mm Durchmesser oder als Split mit einer Teilchengröße von 0,1 bis 0,5 mm oder in wirbelfähiger Form eingesetzt.

Die erfindungsgemäß verwendeten Katalysatoren zeigen auch über einen längeren Zeitraum keine Aktivitätsabnahme. Wenn bei der erfindungsgemäßen Verwendung eine durch Koksabscheidung bedingte Desaktivierung eintritt, empfiehlt es sich, diese durch Abbrennen der Koksablagerung mit Luft oder einem Luft-Stickstoffgemisch, z.B. bei einer Temperatur von 400 bis 550°C zu regenerieren. Andererseits ist es auch möglich durch partielle Verkokung (Prekoke) die Aktivität des Katalysators für ein Selektivitätsoptimum einzustellen. Eine gezielte Beeinflussung der Aktivität und Selektivität läßt sich auch durch eine Vorbehandlung mit H₂S oder schwefelhaltigen organischen Verbindungen bei Temperaturen von 150 bis 300°C, oder eine Imprägnierung mit wäßriger (NH₄)₂S-Lösung erreichen.

Die Spaltung der Formylvaleriansäureester kann diskontinuierlich oder vorteilhaft kontinuierlich als Festbettreaktion mit fest angeordneten Katalysatoren, beispielsweise in Sumpf- oder Rieselfahrweise in der Flüssig- oder Gasphase durchgeführt werden. Es hat sich auch bewährt, die Spaltung als Wirbelbettreaktion in Gegenwart eines sich in auf- und abwirbelnder Bewegung befindlichen Katalysators in der Gasphase durchzuführen.

Eine Ausführungsform in Flüssigphase führt man z.B. so durch, daß man Formylvaleriansäureester und gegebenenfalls Verdünnungsmittel und Sauerstoff enthaltende Gase bei einer Temperatur unterhalb des Siedepunkts des Formylvaleriansäureesters über einen festen Katalysator leitet oder in Gegenwart eines suspendierten Katalysators erhitzt. Das flüssige Reaktionsprodukt wird anschließend nach Abtrennen des Katalysators durch Destillation in Pentenester und nicht umgesetzte Formylvaleriansäureester, die zurückgeführt werden können, getrennt.

Eine bevorzugte Ausführungsform des erfindungsgemäßen Verfahrens in der Gasphase wird beispielsweise durchgeführt, in dem man ein Gemisch aus Formylvaleriansäureester und gegebenenfalls Verdünnungsmittel verdampft und dann zusammen mit Luft und zweckmäßig zusätzlich einem Trägergas wie Stickstoff, Kohlendioxid oder Argon bei der vorgenannten Temperatur gasförmig in eine fest angeordnete oder eine in auf- und abwirbelnder Bewegung befindliche Katalysatorschicht einleitet. Der Reaktionsaustrag wird kondensiert und anschließend durch fraktionierende Destillation aufgetrennt. Nicht umgesetzte Formylvaleriansäureester werden zweckmäßig zurückgeführt.

Nach dem erfindungsgemäßen Verfahren ist es möglich, nicht erwünschte Isomere von Formylvaleriansäureestern wieder in ein Gemisch aus 4-, 3- und 2-Pentensäureestern zu spalten und diese wiederum der Hydroformylierung zuzuführen. Hierdurch gelingt es, Pentensäureester praktisch vollständig in das gewünschte Isomere des 5-Formylvaleriansäureester zu überführen.

5-Formylvaleriansäureeste eignet sich beispielsweise zur Herstellung von Aminocapronsäureester, das zu Caprolactam umgesetzt wird.

Das Verfahren nach der Erfindung sei an folgenden Beispielen veranschaulicht.

### Beispiele 1 bis 16

Die Reaktion wird in der Gasphase unter isothermen Bedingungen in einem Rohrreaktor (Wendel, 0,6 cm Innendurchmesser, 90 cm Länge) mindestens 6 Stunden lang durchgeführt. Die gasförmigen Reaktionsausträge wurden in Kühlfallen kondensiert, gewogen und die Reaktionsprodukte durch übliche Methoden abgetrennt und charakterisiert. Die quantitative Bestimmung der Reaktionsprodukte und der Ausgangsstoffe erfolgte gaschromatographisch. Die Einzelheiten sind nachfolgend ausgeführt.

Für die Beispiele werden folgende Katalysatoren eingesetzt. Es bedeuten PSE: Pentensäureester, FVSE: Formylvaleriansäureester, VSE: Valeriansäureester.

### Katalysator A

Ein Borosilikatzeolith des Pentasil-Typs wird in einer hydrothermalen Synthese aus 640 g hochdispersem SiO₂, 122 g H₃BO₃, 8000 g einer wäßrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% SiO₂ und 2,3 Gew.% B₂O₃. Daraus werden durch Verformen mit einem Verformungshilfsmittel 2 mm-Stränge hergestellt, die bei 110°C/16 h getrocknet und bei 500°C/24 h calciniert werden.

### Katalysator B

Katalysator B wird durch Ionenaustausch des unverstrangten Pulvers von Katalysator A mit einer wäßrigen Pd(NO₃)₂-Lösung erhalten. Nach Trocknung bei 110°C/2 h und Calcination bei 500°C/2 h werden durch Verformen mit einem Verformungshilfsmittel 2 mm-Stränge hergestellt, die bei 110°C/16 h getrocknet und bei 500°C/24 h calciniert werden. Der Palladiumgehalt beträgt 1,1 Gew.% (bezogen auf das Gesamtgewicht).

### Katalysator C

Die Stränge des Borosilikatzeolithen, wie bei Katalysator A beschrieben, werden mit einer ammoniakalischen Palladiumnitrat-Lösung imprägniert. Nach Auswaschen mit H₂O wird bei 110°C getrocknet und bei 500°C/5 h calciniert. Der Pd-Gehalt beträgt 1,2 Gew.%.

### Katalysator D

Katalysator D wird wie Katalysator B hergestellt, jedoch mit einer wäßrigen Lösung aus H₂[PtCl₆] anstatt Pd-Nitrat ausgetauscht. Der Pt-Gehalt beträgt 1,9 Gew.%.

### Katalysator E

Katalysator E wird wie Katalysator C hergestellt, jedoch mit einer wäßrigen Lösung aus RhCl₃ anstatt Pd-Nitrat getränkt. Der Rh-Gehalt beträgt 1,0 Gew.%.

### Katalysator F

Katalysator F wird wie Katalysator C hergestellt, jedoch mit einer wäßrigen Lösung aus Pd- und Na-Nitrat getränkt. Der Pd-Gehalt beträgt 1,0 Gew.%, der Na-Gehalt 0,25 Gew.%.

### Katalysator G

Katalysator G wird wie Katalysator B hergestellt, der Ionenaustausch wird jedoch mit einer wäßrigen Lösung aus Pd- und Zn-Nitrat ausgeführt. Der Pd-Gehalt beträgt 1,1 Gew.%, der Zn-Gehalt 1,0 Gew.%.

### Katalysator H

Katalysator H wird wie Katalysator B hergestellt, der Ionenaustausch wird jedoch mit einer wäßrigen Lösung aus Pd- und Ni-Nitrat ausgeführt. Der Pd-Gehalt beträgt 1,1 Gew.%, der Ni-Gehalt 1,1 Gew.%.

### Katalysator I

Katalysator I wird wie Katalysator B hergestellt, der Ionenaustausch jedoch mit einer wäßrigen Lösung aus RhCl₃ und Co-Nitrat ausgeführt. Der Rh-Gehalt beträgt 1,0 Gew.%, der Co-Gehalt 1,0 Gew.%.

### Katalysator K

Katalysator K wird wie Katalysator B hergestellt, der Ionenaustausch jedoch mit einer wäßrigen Lösung aus RhCl₃ und RuCl₃ ausgeführt. Der Rh-Gehalt beträgt 1,0 Gew.%, der Ru-Gehalt 0,7 Gew.%.

### Katalysator L

Katalysator L wird wie Katalysator B hergestellt, jedoch mit einer wäßrigen Lösung aus RhCl₃, H₂[PtCl₆] und RuCl₃ ausgetauscht. Der Rh-Gehalt beträgt 1,0 Gew.%, der Pt-Gehalt 1,0 Gew.% und der Ru-Gehalt 1,0 Gew.%.

### Katalysator M

Das Siliciumaluminiumphosphat-5 (SAPO-5) wird aus einer Mischung aus 200 g 98 %iger Phosphorsäure, 136 g Boehmit, 60 g Kieselsol (30 %ig), 287 g Tripropylamin und 587 g H₂O hergestellt. Diese Mischung wird bei 150°C während 168 Stunden unter autogenem Druck umgesetzt. Nach Filtration wird das kristalline Produkt bei 120°C getrocknet und bei 500°C calciniert. SAPO-5 enthält 49,8 Gew.% P₂O₅, 33,0 Gew.% Al₂O₃, 6,2 Gew.% SiO₂. SAPO-5 wird mit einem Verstrangungsmittel zu 3 mm-Strängen verformt, bei 120°C getrocknet und bei 500°C calciniert.

Diese Stränge werden mit einer ammoniakalischen Palladiumnitrat-Lösung imprägniert. Nach Auswaschen mit H₂O wird bei 110°C getrocknet und bei 500°C/5 h calciniert. Der Pd-Gehalt beträgt ca. 1 Gew.%.

### Katalysator N

Der Katalysator N ist ein mit Pd dotiertes Aluminiumphosphat, das durch Fällung aus Al(NO₃)₃/H₃PO₄-Lösung mit wäßriger NH₃-Lösung bei pH 6-7 erhalten wird. Nach Abfiltrieren des Niederschlags wird bei 110°C getrocknet und bei 500°C calciniert. Der Al-Gehalt beträgt 28,5 Gew.% und der P-Gehalt 13,2 Gew.%. Das Aluminiumphosphat wird mit einem Verstrangungsmittel zu 3 mm Strängen verformt, bei 120°C getrocknet und bei 500°C calciniert.

Diese Stränge werden mit einer ammoniakalischen Palladiumnitrat-Lösung imprägniert. Nach Auswaschen mit H₂O wird bei 110°C getrocknet und bei 500°C/5 h calciniert. Der Pd-Gehalt beträgt ca. 1 Gew.%.

In der Tabelle I sind für die Beispiele 1 bis 11 der FVSE-, der PSE- und der VSE-Gehalt der Reaktionsausträge nach jeweils 6 h angegeben, (FVSE = Formylvaleriansäuremethylester, PSE = Pentensäuremethylester, VSE = Valeriansäuremethylester).

### Beispiel 1

Pro Stunde wurden ca. 10 ml eines Gemisches aus 4-Formylvaleriansäuremethylester (4-FVSE), Tetrahydrofuran und Wasser (23 Gew.% 4-FVSE, 10 Gew.% Wasser, Rest Tetrahydrofuran) zusammen mit 3 l Stickstoff bei 200°C über 7 g Katalysator G geleitet (Tabelle 1).

### Beispiel 2

Pro Stunde wurden ca. 10 ml eines Gemisches aus 4-FVSE, Methanol und Wasser (23 Gew.% 4-FVSE, 10 Gew.% Wasser, Rest Methanol) zusammen mit 3 l Stickstoff bei 200°C über 7 g Katalysator G geleitet (Tabelle 1).

### Beispiel 3

Pro Stunde wurden ca. 10 ml eines Gemisches aus 4-Formylvaleriansäuremethylester (4-FVSE). Tetrahydrofuran und Wasser (23 Gew.% 4-FVSE, 10 Gew.% Wasser, Rest Tetrahydrofuran) zusammen mit 1 l Luft und 2 l Stickstoff bei 200°C über 7 g Katalysator G geleitet (Tabelle 1).

### Beispiel 4

Pro Stunde wurden ca. 10 ml eines Gemisches aus 4-FVSE, Methanol und Wasser (23 Gew.% 4-FVSE, 10 Gew.% Wasser, Rest Methanol) zusammen mit 1 l Luft und 2 l Stickstoff bei 200°C über 7 g Katalysator G geleitet (Tabelle 1).

### Beispiele 5 bis 7

Pro Stunde wurden ca. 10 ml eines Gemisches aus 4-FVSE, Methanol und Wasser (23 Gew.%` 4-FVSE, 10 Gew.% Wasser, Rest Methanol) zusammen mit 1 l Luft und 2 l Stickstoff bei 200°C über 7 g Katalysator B, E bzw. I geleitet (Tabelle 1).

### Beispiele 8 und 9

Pro Stunde wurden ca. 10 ml eines Gemisches aus 4-FVSE, 3-FVSE, Methanol und Wasser (14,5 Gew.-% 4-FVSE, 7,4 Gew.-% 3-FVSE, 10 Gew.-% Wasser. Rest Methanol) zusammen mit 3 l Luft bei 185°C über 7 g Katalysator C bzw. F geleitet (Tabelle 1).

### Beispiele 10 und 11

Pro Stunde wurden ca. 10 ml eines Gemisches aus 4-FVSE, Methanol und Wasser (23 Gew.-% 4-FVSE, 10 Gew.-% Wasser, rest Methanol) zusammen mit 1 l Luft und 2 l Stickstoff bei 200°C über 7 g Katalysator K bzw. L geleitet (Tabelle 1).

**Tabelle I**

| Beispiel | 1 | 2 | 3 | 4 | 5 | 6 | 7 | 8 | 9 | 10 | 11 |
|---|---|---|---|---|---|---|---|---|---|---|---|
| Katalysator | G | G | G | G | B | E | I | C | F | K | L |
| Temperatur | 200°C | 200°C | 200°C | 200°C | 200°C | 200°C | 200°C | 185°C | 185°C | 200°C | 200°C |
| WHSV h⁻¹ | 1,2 | 1,2 | 1,2 | 1,2 | 1,1 | 1,1 | 1,2 | 1,0 | 1,2 | 1,2 | 1,2 |
| FVSE F1-% | 65 | 33 | 79 | 2 | - | - | 4 | - | - | 20 | - |
| PSE F1-% | 21 | 21 | 14 | 65 | 79 | 55 | 62 | 79 | 69 | 47 | 56 |
| VSE F1-% | 5 | 12 | 2 | 24 | 13 | 31 | 21 | 4 | 2 | 11 | 32 |

### Beispiel 12

In einem Langzeitversuch über 122 h wurden pro Stunde ca. 10 ml eines Gemisches aus 4-FVSE, Methanol und Wasser (23 Gew.-% 4-FVSE, 10 Gew.-% Wasser, Rest Methanol) zusammen mi 1 l Luft und 1 l Stickstoff bei 200°C über 7 g Katalysator G geleitet. In der Tabelle 2 sind der FVSE-, der PSE- und der VSE-Gehalt der Reaktionsausträge nach der jeweiligen Versuchsdauer angegeben.

**Tabelle 2**

| Nr. | Versuchsdauer (h) | PSE (F1-%) | VSE (F1-%) | FVSE (F1-%) |
|---|---|---|---|---|
| 1 | 6 | 71 | 16 | 2 |
| 2 | 22 | 73 | 19 | 1 |
| 3 | 46 | 72 | 18 | 1 |
| 4 | 76 | 67 | 24 | 2 |
| 5 | 102 | 69 | 21 | 2 |
| 6 | 122 | 71 | 22 | 1 |

Nach 122 h Versuchsdauer wurden die vereinigten Reaktionsausträge quantitativ analysiert:
- Austrag: 695 g: Pentensäuremethylester/Valeriansäuremethylester/Solvenz`Gemisch (12 Gew'% Pentenester (8 % 4-, 50 % 3- und 42 % 2-trans-Pentensäuremethylester)/5 Gew.-% Valerianester + 2-cis-PSE)

### Beispiel 13

In einem Langzeitversuch über 30 h wurden pro Stunde 11 g eines 4-FVSE und 3-FVSE enthaltenden Gemisches (63 % 4-FVSE, 32 Gew'-% 3-FVSE) in einen Verdampfer gepumpt und von dort zusammen mit 3 l Luft und 7 l Stickstoff bei 200°C über 7 g Katalysator F geleitet. Die gasförmigen Reaktionsausträge wurden nach 30 h Versuchsdauer gewogen und gaschromatographisch analysiert (Gesamteinsatz: 336 g FVSE). In der Tabelle 3 sind der FVSE-, der PSE- und der VSE-Gehalt der Reaktionsausträge nach der jeweiligen Versuchsdauer angegeben.

**Tabelle 3**

| Nr. | Versuchsdauer (h) | PSE (F1-%) | VSE (F1-%) | FVSE (F1-%) |
|---|---|---|---|---|
| 1 | 6 | 59 | 18 | 19 |
| 2 | 11 | 61 | 18 | 17 |
| 3 | 19 | 60 | 16 | 20 |
| 4 | 27 | 54 | 12 | 30 |

Nach 30 h Versuchsdauer wurden die vereinigten Reaktionsausträge quantitativ analysiert:
- Austrag: 312 g: Pentensäuremethylester/Valeriansäuremethylester/Solvenz-Gemisch (46 Gew.% Pentenester (13 % 4-, 65 % 3- und 22 % 2-trans-Pentensäuremethylester)/16 Gew.-% Valerianester + 2-cis-PSE)
63 % Umsatz, 85 % Selektivität zu Pentenester

### Beispiel 14

Innerhalb 6 Stunden wurden stündlich 12 g eines 4-FVSE und 3-FVSE enthaltenden Genisches (63 % 4-FVSE, 32 X 3-FVSE) in einen Verdampfer gepumpt und von dort zusammen mit 8 l Luft und 3 l Stickstoff bei 185°C über 7 g Katalysator C geleitet. Die kondensierten Reaktionsausträge wurden nach 6 h Versuchsdauer gewogen und gaschromatographisch analysiert (Tabelle 4).

### Beispiel 15

Über einen Zeitraum von 6 Stunden wurden stündlich 11 g eines 4-FVSE und 3-FVSE enthaltenden Gemisches (63 % 4-FVSE, 32 % 3-FVSE) in einen Verdampfer gepumpt und von dort zusammen mit 6 l Luft und 5 l Stickstoff bei 185°C Ober 7 g Katalysator M geleitet. Die kondensierten Reaktionsausträge wurden nach 6 h Versuchsdauer gewogen und gaschromatographisch analysiert (Tabelle 4).

### Beispiel 16

Über einen Zeitraum von 6 Stunden wurden stündlich 11 g 5-FVSE in einen Verdampfer gepumpt und von dort zusammen mit 11 l Stickstoff bei 185°C über 7 g Katalysator F geleitet. Die kondensierten Reaktionsausträge wurden nach 6 h Versuchsdauer gewogen und gaschromatographisch analysiert (Tabelle 4).

**Tabelle 4**

| Bsp. | Kontakt | Einsatzgemisch | PSE (Gew%) | VSE (Gew.%) | FVSE (Gew.%) | Umsatz % | Selektivität zu PSE % |
|---|---|---|---|---|---|---|---|
| 14 | C | 3/4-FVSE | 70 | 10 | 13 | 88 | 88 |
| 15 | M | 3/4-FVSE | 25 | 5 | 50 | 45 | 65 |
| 16 | F | 5-FVSE | 30 | 16 | 45 | 65 | 45 |

## Patentansprüche

1. Verfahren zur Herstellung von Pentensäureestern, dadurch gekennzeichnet, daß man 3- oder 4-Formylvaleriansäureester oder Mischungen aus 50 bis 80 Gew.% 4-Formylvaleriansäureestern, 20 bis 40 Gew.-% 3-Formylvaleriansäureestern und bis zu 10 Gew.% 5-Formylvaleriansäureestern in Gegenwart von Zeolithen oder mindestens einem Phosphat, wobei die Zeolithe oder Phosphate einen Gehalt an mindestens einem Metall der VIII. Nebengruppe des periodischen Systems haben, ausgewählt aus der Gruppe Alumiuniumphosphat, Siliciumaluminiumphosphat, Boraluminiumphosphat, Siliciumeisenaluminiumphosphat, Cerphosphat, Zirkonphosphat, Borphosphat, Eisenphosphat und Strontiumphosphat auf eine Temperatur von 50 bis 400°C erhitzt.

2. Verfahren nach Anspruch 1, dadurch gekennzeichnet, daß man molekularen Sauerstoff oder solchen enthaltende Gase mitverwendet.

3. Verfahren nach den Ansprüchen 1 und 2, dadurch gekennzeichnet, daß man unter Reaktionsbedingungen inerte verdünnungsmittel mitverwendet.

4. Verfahren nach den Ansprüchen 1 bis 3, dadurch gekennzeichnet, daß die Zeolithe oder Phosphate einen Gehalt an mindestens einem Edelmetall der VIII. Gruppe des Periodischen Systems haben.

5. Verfahren nach den Ansprüchen 1 bis 4, dadurch gekennzeichnet, daß die Zeolithe oder Phosphate einen Gehalt an mindestens einem Edelmetall der VIII. Nebengruppe des Periodischen Systems und zusätzlich einen Gehalt an mindestens einem Metall der Eisengruppe haben.

6. Verfahren nach den Ansprüchen 1 bis 5, dadurch gekennzeichnet, daß die Zeolithe oder Phosphate zusätzlich einen Gehalt an mindestens einem Element der I. bis VII. Nebengruppe des Periodischen Systems haben.

7. Verfahren nach den Ansprüchen 1 bis 6, dadurch gekennzeichnet, daß man Zeolithe mit Pentasilstruktur verwendet.

8. Verfahren nach den Ansprüchen 1 bis 7, dadurch gekennzeichnet, daß man Aluminiumsilikatzeolith, Borsilikatzeolith oder Eisensilikatzeolith mit Pentasilstruktur verwendet.

9. Verfahren nach den Ansprüchen 1 bis 8, dadurch gekennzeichnet, daß man Aluminiumsilikatzeolith mit Faujasit-Struktur verwendet.

10. Verfahren nach den Ansprüchen 1 bis 8, dadurch gekennzeichnet, daß man Aluminiumphosphat, Siliciumaluminiumphosphat, Eisenaluminiumphosphat oder Boraluminiumphosphat verwendet.

## Claims

1. A process for preparing a pentenoic ester, which comprises heating a 3- or 4-formylvaleric ester or a mixture of from 50 to 80% by weight of a 4-formylvaleric ester, from 20 to 40% by weight of a 3-formylvaleric ester and up to 10% by weight of a 5-formylvaleric ester at from 50 to 400°C in the presence of a zeolite or one or more phosphates containing one or more metals of group VIII of the periodic table, selected from the group consisting of aluminum phosphate, silicon aluminum phosphate, boron aluminum phosphate, silicon iron aluminum phosphate, cerium phosphate, zirconium phosphate, boron phosphate, iron phosphate and strontium phosphate.

2. A process as claimed in claim 1, wherein molecular oxygen or a gas containing same is present.

3. A process as claimed in claims 1 and 2, wherein a diluent which is inert under the reaction conditions is present.

4. A process as claimed in any of claims 1 to 3, wherein the zeolite or phosphate contains one or more noble metals of group VIII of the periodic table.

5. A process as claimed in any of claims 1 to 4, wherein the zeolite or phosphate contains one or more noble metals of group VIII of the periodic table and additionally one or more metals of the iron group.

6. A process as claimed in any of claims 1 to 5, wherein the zeolite or phosphate additionally contains one or more elements of groups I-VII of the periodic table.

7. A process as claimed in any of claims 1 to 6, wherein a zeolite having a pentasil structure is used.

8. A process as claimed in any of claims 1 to 7, wherein aluminum silicate zeolite, boron silicate zeolite or iron silicate zeolite having a pentasil structure is used.

9. A process as claimed in any of claims 1 to 8, wherein aluminum silicate zeolite having a faujasite structure is used.

10. A process as claimed in any of claims 1 to 8, wherein aluminum phosphate, silicon aluminum phosphate, iron aluminum phosphate or boron aluminum phosphate is used.

## Revendications

1. Procédé de préparation d'esters d'acides penténoïques, caractérisé en ce que l'on chauffe, à une température de 50-400°C, de l'ester d'acide 3- ou 4-formylvalérianique ou des mélanges formés de 50-80% en poids d'esters d'acide 4-formylvalérianique, de 20-40% en poids d'esters d'acides 3-formylvalérianique et de jusqu'à 10% en poids d'esters d'acide 5-formylvalérianique, en présence de zéolites ou d'au moins un phosphate, les zéolites ou les phosphates contenant au moins un métal du groupe VIII de la classification périodique des éléments, choisi dans le groupe formé par les phosphates d'aluminium, les phosphates de silicium et d'aluminium, les phosphates de bore et d'aluminium, les phosphates de silicium, de fer et d'aluminium, les phosphates de cérium, les phosphates de zirconium, les phosphates de bore, les phosphates de fer et les phosphates de strontium.

2. Procédé selon la revendication 1, caractérisé en ce que l'on utilise de l'oxygène moléculaire ou des gaz en contenant.

3. Procédé selon la revendication 1 ou 2, caractérisé en ce que l'on utilise conjointement des agents diluants inertes dans les conditions réactionnelles.

4. Procédé selon l'une quelconque des revendications 1 à 3, caractérisé en ce que les zéolites ou les phosphates contiennent au moins un métal rare du groupe VIII de la classification périodique des éléments.

5. Procédé selon l'une quelconque des revendications 1 à 4, caractérisé en ce que les zéolites ou les phosphates contiennent au moins un métal rare du groupe VIII de la classification périodique des éléments, et, en outre, au moins un métal du groupe du fer.

6. Procédé selon l'une quelconque des revendications 1 à 5, caractérisé en ce que les zéolites ou les phosphates contiennent en outre au moins un élément des groupes IB à VIII de la classification périodique des éléments.

7. Procédé selon l'une quelconque des revendications 1 à 6, caractérisé en ce que l'on utilise des zéolites à structure pentasile.

8. Procédé selon l'une quelconque des revendications 1 à 7, caractérisé en ce que l'on utilise des zéolites aluminosilicates, borosilicates ou ferrosilicates à structure pentasile.

9. Procédé selon l'une quelconque des revendications 1 à 8, caractérisé en ce que l'on utilise des zéolites aluminosilicates, à structure de faujasite.

10. Procédé selon l'une quelconque des revendications 1 à 8, caractérisé en ce que l'on utilise des phosphates d'aluminium, des phosphates de silicium et d'aluminium, des phosphates de fer et d'aluminium ou des phosphates de bore et d'aluminium.
